# EUROPEAN PATENT APPLICATION

(11) **EP 4 487 757 A1**
(43) Date of publication of application: **08.01.2025**
(21) Application number: 22929987.0
(22) Date of filing: 02.03.2022
(51) Int. Cl.: A61B 5/00, A61B 8/06, A61B 8/12, A61B 8/08, A61B 5/0295, A61B 5/026, A61B 18/14, A61B 18/00

(54) **METHOD FOR EVALUATING OUTCOME OF RENAL DENERVATION**

(71) Applicant: Park, Eul Joon, Seoul 06356 (KR)
(72) Inventor: TSIOUFIS, Konstantinos, 15236 Athens (GR); PARK, Eul Joon, Seoul 06356 (KR)
(74) Representative: Forrest, Stuart
(86) International application number: PCT/KR2022/002901
(87) International publication number: WO 2023/167343

(57) **Abstract**

According to an embodiment disclosed herein, a method for evaluating the outcome of renal denervation comprises the steps of: acquiring first blood flow data from a first region in a renal blood vessel after a vasodilator injected into the first region is activated in a second region in the renal blood vessel; and acquiring second blood flow data from the first region after renal denervation is performed with a catheter for denervation. In addition, according to an embodiment disclosed herein, a device for evaluating the outcome of renal denervation comprises: a first input unit for inputting first blood flow data acquired from a first region in a renal blood vessel after a vasodilator injected into the first region is activated in a second region; and a second input unit for inputting second blood flow data acquired from the first region after renal denervation is performed with a catheter.

## Description

### TECHNICAL FIELD

Embodiments relate to a method and device for evaluating outcomes of renal denervation, more particularly, to a method and device for evaluating the outcomes of renal denervation that easily evaluates whether the renal denervation of the sympathetic nerve is performed after injection of a vasodilator.

### BACKGROUND ART

Abnormally high sympathetic nerve activation exists in patients with chronic diseases such as hypertension, kidney disease, heart failure, diabetes, etc. In the case of resistant hypertension, which accounts for 20 to 30% of all hypertensive patients, blood pressure is not controlled below a target level even after treatment with three or more blood pressure lowering drugs, including diuretics. Therefore, to treat chronic diseases, sympathetic modulation such as renal sympathetic depression has been used.

A representative treatment for resistant hypertension is nerve ablation, which is a surgical procedure that includes cutting the renal nerves that transmit signals to the Renin-Angiotensin-Aldosterone System (RAAS), one of the important mechanisms related to blood pressure control, and lowering blood pressure by reducing nervous system activity. In this case, the renal sympathetic nerves exist in the subdermal layer of the renal artery, and the afferent and efferent sympathetic nerves also exist in the renal artery, so renal denervation for the treatment of hypertension is performed in the renal artery.

Meanwhile, it needs to check whether the renal denervation has been sufficiently achieved through the above-described renal denervation procedure and, if insufficient, to ensure renal denervation through re-treatment. However, if too much time is required to check and evaluate the outcomes during the procedure, or if the patient is made to wait for a long time, there is a problem that not only reduces the effect of the procedure, but also makes the patient suffer. Therefore, it is important to quickly and accurately confirm and evaluate the outcomes of renal denervation procedures.

### DISCLOSURE

### TECHNICAL PROBLEM

In a method and device for evaluating an outcome of renal denervation according to the present invention, a vasodilator (e.g. dopamine, dobutamine, adenosine, prostacyclin, nitric oxide, etc.) is injected into a renal vasculature, but the direct effect on the renal artery is minimized, and the injection location of the vasodilator is determined so that the vasodilator is activated in the renal arterioles or renal capillaries (commonly referred to as the 'renal arteriole') connected to the renal artery. Accordingly, the present invention provides a method and device for evaluating an outcome of renal denervation, which acquires data on blood flow within the renal artery in a state of maximal vasodilation of the renal arteriole and performs an accurate evaluation of renal denervation.

In other words, in the method and device for evaluating an outcome of renal denervation according to the present invention, a vasodilator is injected into a specific location in the renal artery (i.e., first region), the residence time of the vasodilator in the renal artery is minimized, so that the vasodilator is mainly activated in the renal arterioles (i.e., second region) without significantly affecting the diameter or size of renal artery vessels. Accordingly, the renal denervation is evaluated using blood flow data obtained through dilated renal arterioles.

In this case, when the vasodilator is injected into a specific location of the renal artery, the renal arterioles may be dilated to the maximum blood vessel level without affecting the vasodilation of the renal artery. Also, in order to accurately measure blood flow in the renal artery, blood flow must be measured in the activated state. Here, blood flow refers to the amount of blood flowing through blood vessels per unit time. The blood flow in the renal artery must be measured while minimizing the effect of vasodilators on the renal artery and maximally dilating the blood vessels in the renal arterioles in order to accurately measure the maximum blood flow unique to each patient.

Accordingly, the method and device for evaluating an outcome of renal denervation according to the present invention may acquire blood flow data in the same state as the state in which the sympathetic nerve is ablated from the renal artery.

Accordingly, a method for evaluating an outcome of renal denervation compares the maximum blood flow data in the renal artery (first blood flow data), which is unique to each patient, with the blood flow data in the renal artery (second blood flow data) acquired after renal denervation by catheter has been performed, so the evaluation and determination on the renal denervation may be performed quickly and accurately, minimizing the burden on patients.

In addition, based on the comparison results between the blood flow data (first and second blood flow data), if the comparison results are greater than or equal to a predetermined threshold value, it may be determined that the procedure has been successful, and if the comparison results are lower than or equal to a predetermined value, it may be determined that the procedure has not been performed properly, so reoperation is necessary.

In addition, the method and device for evaluating an outcome of renal denervation according to an embodiment may acquire information about blood flow in the renal artery in the state of maximum vasodilation by injecting a vasodilator into the renal vasculature before renal denervation. In other words, the method and device for evaluating an outcome of renal denervation may acquire blood flow information that is not affected by the compound factors of blood vessels that occur after renal denervation. For example, compound factors in blood vessels may include muscle cramps (spasm) due to stimulation of the ache nerve after nerve ablation, changes in blood pressure due to stimulation of the sympathetic nerve, etc. In other words, if information on blood flow in the state of maximum vasodilation is acquired by injecting a vasodilator into the renal blood vessels after renal denervation, the results will be different from those before renal denervation, making it impossible to acquire accurate comparison value information.

In addition, the method and device for evaluating an outcome of renal denervation according to another embodiment of the present invention may quickly and accurately perform evaluation and determination on the renal denervation through a catheter by using not only the above-mentioned content but also patient's other usual blood flow data depending on the patient's physical condition or disease stage.

Specifically, the method and device for evaluating an outcome of renal denervation according to another embodiment acquire the patient's usual blood flow data (base blood flow data). Thus, even if the usual blood flow data is different for each patient, the first blood flow data and second blood flow data are respectively compared based on the usual blood flow data for each patient, so evaluation of renal denervation may be accurately performed for each patient.

Further, the method and device for evaluating an outcome of renal denervation according to the present invention compare a ratio of the differences between the usual blood flow data and each of the above-mentioned first and second blood flow data with a threshold value, and thus, may easily and accurately determine whether the renal denervation procedure has been successful for each patient. In addition, if the ratio is lower than or equal to a predetermined threshold value, this is a result of the procedure not being performed properly. Thus, the method and device for evaluating an outcome of renal denervation may easily determine whether to reperform the procedure.

Furthermore, by acquiring the patient's usual blood flow data (base blood flow data) before administering the vasodilator, changes in blood flow data due to the vasodilator or renal denervation may be eliminated. As a result, it is possible to acquire reference data (usual blood flow data) for more accurate evaluation of an outcome of renal denervation.

The objects to be achieved in the embodiment are not limited thereto, and it can be said that the objects and effects that may be understood from the means of solving the problems and embodiments described below are also included.

### TECHNICAL SOLUTION

A method for evaluating an outcome of renal denervation according to an embodiment comprises the steps of acquiring first blood flow data from a first region in a renal blood vessel after a vasodilator injected into the first region is activated in a second region in the renal blood vessel; and acquiring second blood flow data from the first region after renal denervation is performed with a catheter for denervation.

The method may further comprise the step of comparing the first blood flow data and the second blood flow data.

The renal denervation may be evaluated according to a result of the comparison of the first blood flow data and the second blood flow data.

If the comparison result is lower than a predetermined threshold, the step of re-performing renal denervation may be repeated.

The method may further comprise the steps of calculating a first difference value representing a difference value between the first blood flow data and base blood flow data; calculating a second difference value representing a difference between the second blood flow data and the base blood flow data; and comparing the first difference value and the second difference value.

An outcome of the renal denervation may be evaluated according to a result of the comparison of the first difference value and the second difference value.

If the comparison result is lower than a predetermined threshold, the step of re-performing renal denervation may be repeated.

The base blood flow data may be acquired before injecting the vasodilator, or between the step of acquiring the first blood flow data and the step of performing the renal denervation.

Base blood flow data or the second blood flow data may be acquired when a half-life has elapsed after the vasodilator is injected into the first region.

The second region may be a renal arteriole or renal capillary.

The vasodilator may include any one of dopamine, dobutamine, adenosine, prostacyclin, nitric oxide, bradykinin, papaverine, dipyridamolum, diuretin, theophylline, minoxidil, and isosorbide.

A device for evaluating an outcome of renal denervation according to an embodiment may be configured to comprise a first input unit that inputs first blood flow data acquired from a first region in a renal blood vessel after a vasodilator injected into the first region is activated in a second region; and a second input unit that inputs second blood flow data acquired from the first region after renal denervation is performed with a catheter.

The device may further comprise a first calculation unit that calculates a ratio between the first blood flow data input by the first input unit and the second blood flow data input by the second input unit.

The device may further comprise an evaluation unit that evaluates an outcome of renal denervation by comparing the ratio between the first blood flow data and the second blood flow data calculated by the first calculation unit with a first threshold value.

The device may further comprise a third input unit that inputs base blood flow data from the first region before injection of the vasodilator.

The device may further comprise a second calculation unit that calculates a ratio between a first difference value and a second difference value, wherein the first difference value may be a difference value between the first blood flow data and the base blood flow data, and the second difference value may be a difference value between the second blood flow data and the base blood flow data.

The device may further comprise an evaluation unit that evaluates the outcome of renal denervation by comparing the ratio between the first difference value and the second difference value calculated by the second calculation unit with a second threshold value.

The device may further comprise a display unit that displays an evaluation result of the evaluation unit.

The device may further comprise an output unit that outputs an audio signal or video signal, etc., according to the evaluation result of the evaluation unit.

The first blood flow data, the second blood flow data, and the base blood flow data are acquired from blood flow information detected by a detection means disposed on the catheter.

The blood flow information may include at least one of blood flow volume, blood flow rate, and blood flow resistance.

The detection means may include any one of an ultrasonic sensor, an impedance sensor, and an optical sensor.

The ultrasonic sensor may include a Doppler sensor.

An impedance value may be detected by an electrode of the catheter or detected by a separate sensor.

The catheter may include a vasodilator injection unit.

### ADVANTAGEOUS EFFECTS

According to an embodiment, it is possible to acquire intravascular blood flow data of the renal artery immediately after renal denervation, thereby providing a method and device for evaluating an outcome of renal denervation capable of reducing a procedure time.

In addition, by comparing the maximum blood flow data unique to each patient with the blood flow data after performing renal denervation, it is possible to provide a method and device for evaluating an outcome of renal denervation capable of more accurately performing patient-specific evaluation of the nerve denervation procedure.

In addition, it is possible to provide a method and device for evaluating an outcome of renal denervation capable of reducing the patient's physical burden by reducing the procedure time, including evaluation of an outcome of renal denervation.

In addition, it is possible to provide a method and device for evaluating an outcome of renal denervation capable of directly expanding the blood vessels of the renal artery.

In addition, the present invention may provide a method and device for evaluating an outcome of renal denervation that may quickly and accurately perform evaluation and determination on the renal denervation through a catheter by additionally using patient's other usual blood flow data depending on the patient's physical condition or disease stage. In other words, it is possible to provide a method and device for evaluating an outcome of renal denervation capable of accurately evaluating an outcome of renal denervation for each patient even if each patient's condition is different.

The various and beneficial advantages and effects of the present invention are not limited to the above-described content, and may be more easily understood through description of specific embodiments of the present invention.

### DESCRIPTION OF DRAWINGS

FIG. 1 is a schematic diagram of a renal denervation system according to an embodiment.
FIG. 2 is a diagram illustrating a method for evaluating an outcome of renal denervation according to an embodiment.
FIG. 3 is a perspective view schematically illustrating a configuration of a distal end side of a catheter according to the embodiment.
FIG. 4 is a diagram illustrating a portion of FIG.
FIG. 5 is a flowchart of a method for evaluating an outcome of renal denervation according to an embodiment.
FIG. 6 is a flowchart of a method for evaluating an outcome of renal denervation according to another embodiment.
FIG. 7 is a flowchart of a method for evaluating an outcome of renal denervation according to another embodiment.
FIG. 8 is a diagram explaining a method of acquiring base blood flow data in a method for evaluating an outcome of renal denervation according to the present invention.
FIG. 9 is a diagram explaining an example of a method of acquiring blood flow data.
FIG. 10 is a diagram explaining another example of a method of acquiring blood flow data.
FIG. 11 is a diagram explaining another example of a method of acquiring blood flow data.
FIG. 12 is a diagram explaining injection of a vasodilator in a method for evaluating an outcome of renal denervation according to an embodiment.
FIG. 13 is a diagram explaining a method of acquiring first blood flow data in a method for evaluating an outcome of renal denervation according to an embodiment.
FIG. 14 is a graph of an action of a vasodilator in a method for evaluating an outcome of renal denervation according to an embodiment.
FIG. 15 is a diagram explaining renal denervation using a catheter and acquiring second blood flow data in a method for evaluating an outcome of renal denervation according to an embodiment.
FIG. 16 is a flowchart explaining comparison of first blood flow data and second blood flow data in a method for evaluating an outcome of renal denervation according to an embodiment.
FIG. 17 is a block diagram of a device for evaluating an outcome of renal denervation according to an embodiment.

### MODE FOR INVENTION

The present invention may have various modifications and various exemplary embodiments and specific exemplary embodiments will be illustrated in the drawings and described. However, this does not limit the present invention to specific exemplary embodiments, and it should be understood that the present invention covers all the modifications, equivalents and replacements within the idea and technical scope of the present invention.

Terms including an ordinal number such as first or second may be used to describe various components but the components are not limited by the above terms. The above terms are used only to discriminate one component from the other component. For example, without departing from the scope of the present invention, a second component may be referred to as a first component, and similarly, the first component may be referred to as the second component. A terminology such as and/or includes a combination of a plurality of associated items or any item of the plurality of associated items.

It should be understood that, when it is described that an element is "coupled" or "connected" to another element, the element may be "directly coupled" or "directly connected" to the other element or "coupled" or "connected" to the other element through a third element. In contrast, it should be understood that, when it is described that an element is "directly coupled" or "directly connected" to another element, it is understood that no element is not present between the element and the other element.

Terms used in the present application are used only to describe specific exemplary embodiments, and are not intended to limit the present invention. A singular form may include a plural form if there is no clearly opposite meaning in the context. In the present application, it should be understood that term "include" or "have" indicates that a feature, a number, a step, an operation, a component, a part or the combination thereof described in the specification is present, but does not exclude a possibility of presence or addition of one or more other features, numbers, steps, operations, components, parts or combinations, in advance.

If it is not contrarily defined, all terms used herein including technological or scientific terms have the same meaning as those generally understood by a person with ordinary skill in the art. It should be understood that terms defined in a generally used dictionary have the same meanings as contextual meanings of associated techniques and if not apparently defined in this application, the terms should not be interpreted as ideological or excessively formal meaning.

Hereinafter, exemplary embodiments of the present invention will be described in detail with reference to the accompanying drawings, in which like reference numerals refer to like or similar elements regardless of reference numerals and a duplicated description thereof will be omitted.

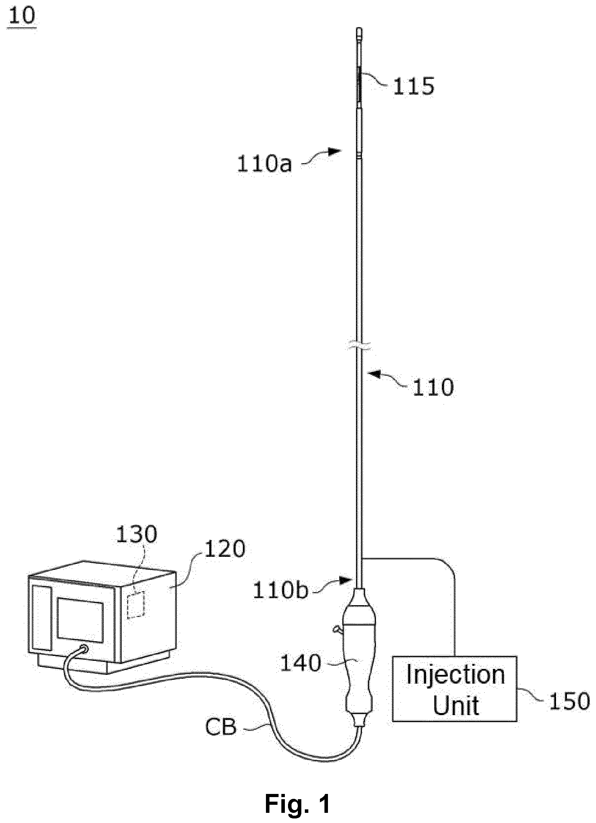

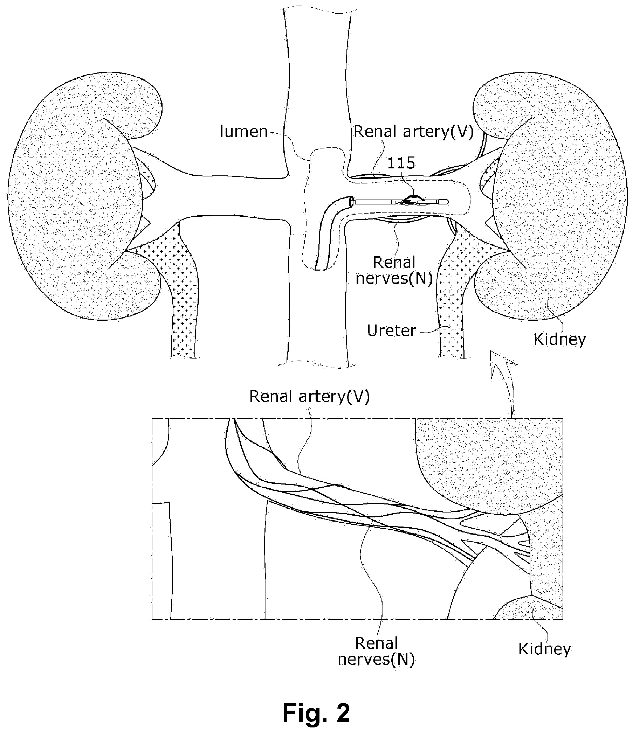

FIG. 1 is a schematic diagram of a renal denervation system according to an embodiment, and FIG. 2 is a diagram explaining a renal denervation method according to an embodiment.

Referring to FIGS. 1 and 2, a renal denervation system 10 according to the embodiment may include a catheter 110, a console 120, a renal denervation outcome evaluation device 130, a handle 140, and an injection unit 150.

The catheter 110 is connected to the console 120 through a cable CB and may include a plurality of electrodes 115 at the distal end 110a. This catheter 110 may be moved to a blood vessel (e.g., artery) connected to an organ (e.g., kidney or ureter).

In addition, when the plurality of electrodes 115 in the catheter 110 are located within a blood vessel V (for example, a renal artery), energy may be transmitted to the electrode 115 in contact with the wall of the blood vessel V according to the manipulation of the user or operator through the handle 140 (e.g., energy transfer that generates high temperature). In addition, nerves (hereinafter referred to as renal nerves) may be located outside the blood vessels along the arteries. Hereinafter, the lumen of the artery and renal artery will be partially illustrated and explained as shown in the drawings. Through this, energy may also be transmitted to nerves outside the wall of the blood vessel V that are connected to or adjacent to the wall of the blood vessel V. For example, heat energy transmitted through the plurality of electrodes 115 of the catheter 110 is transmitted to the outer or adjacent nerves (e.g., renal nerves) of the blood vessel V, and the renal denervation may occur due to the transmitted energy. Hereinafter, the description will be based on the assumption that renal denervation is performed by the above-described method.

The console 120 may control and monitor the operation of the catheter 110 so that the above-described renal denervation (e.g., renal denervation ablation) is performed. For example, the console 120 may be configured to continuously or intermittently monitor blood flow data (e.g., blood flow rate, blood flow volume) within the blood vessel V. For example, a renal denervation outcome evaluation device 130 may be mounted in the console 120. However, the present invention is not limited thereto, and the renal denervation outcome evaluation device 130 may be implemented as a handle or single external device. Hereinafter, the description will be made based on the renal denervation outcome evaluation device 130 being mounted within the console 120.

The console 120 may provide the user with blood flow data in the blood vessel V or data on a degree of renal denervation performed in a target region through the renal denervation outcome evaluation device 130. For example, when it is determined that the renal denervation has not been performed properly or when it is determined that the renal denervation has properly performed, the renal denervation outcome evaluation device 130 may provide the user with data (e.g., alarm) corresponding to the evaluation of the renal denervation.

In addition, the console 120 may adjust the size and form of energy provided to the electrode 115. For example, the console 120 may vary the size and form of energy depending on the target of renal denervation, etc.

In addition, in the case of electrode-based or heat transfer-based, console 120 may include or be connected to an energy generator that generates energy (e.g., ultrasound, heat), for example. Furthermore, the energy generator may provide various types of energy, such as pulse energy, ultrasonic energy, microwave energy, optical energy, heat energy, and radiation.

As described above, the renal denervation outcome evaluation device 130 may be located within the console 120 and may determine the degree of renal denervation performance and provide a corresponding alarm to the user. A detailed explanation of this will be provided later.

The handle 140 is located between the console 120 and the catheter 110 and may be connected to the console 120 and catheter 110. In an embodiment, the handle 140 may contact the proximal end 110b of the catheter 110.

In addition, the handle 140, like the console 120, may be located outside the patient and may include an operating unit for ease of operation by the user. For example, the control unit may be implemented in various ways, such as buttons and jogs.

In addition, the user may control the operation of the catheter 110, etc. through the handle 140. That is, when a command corresponding to the user's manipulation is generated, the generated command may be transmitted to the console 120 or catheter 110, and operation corresponding to the command may be performed in the console 120 or catheter 110. For example, energy is transferred to the electrode 115 of the catheter 110 by a user's manipulation (e.g., turning on an energy injection button), and renal denervation may be performed with the transferred energy. For example, by manipulating or driving the handle 140 by the user or operator, the electrodes of the catheter may be extended or de-extended into the outer or renal blood vessels. For example, when inserting the distal end 11a of the catheter into a blood vessel, the electrodes of the catheter may have a spine structure. The spine structure of this electrode may be inserted into a blood vessel in a non-expanded state (the spine structure is spread out in a straight line) depending on the manipulation of the user or operator.

Further, after the distal end of the catheter is inserted into the renal artery, the spine structure of the electrode may be expanded by the user manipulating the handle 140 so that the electrode of the catheter makes good contact with the blood vessel wall when acquiring blood flow data or performing renal denervation (where the spine structure may be bent into a '⊏' shape, for example). Furthermore, as another example, the electrodes of the catheter may be attached to the spine structure. In the present invention, it may be understood that the catheter has the spine structure so that the electrode of the catheter easily contacts the blood vessel wall.

An injection unit 150 may be connected to the catheter 110. In addition, the injection unit 150 may be coupled to the proximal end 110b of the catheter 110 to inject fluid into the catheter 110. In an embodiment, the catheter 110 may deliver fluid (e.g., indicator solution) to the blood vessel V. Accordingly, the indicator solution may be injected into a region close to the distal end 110a of the catheter 110. In addition, the catheter 110 may include various ports for injecting the indicator solution. Alternatively, the catheter 110 may further include another guide member and be combined with the guide member to deliver the indicator solution. Alternatively, the indicator solution may be delivered to the target through another delivery member that is distinct from the catheter 110.

In addition, the injection unit 150 may contain a fluoroscopic contrast agent for use in imaging. Alternatively, the injection unit 150 may be made of various materials capable of identifying IVUS, OCT, and target treatment sites. In addition, the injection unit 150 may deliver a treatment substance or vasodilator to a target, including a treatment substance or vasodilator described later.

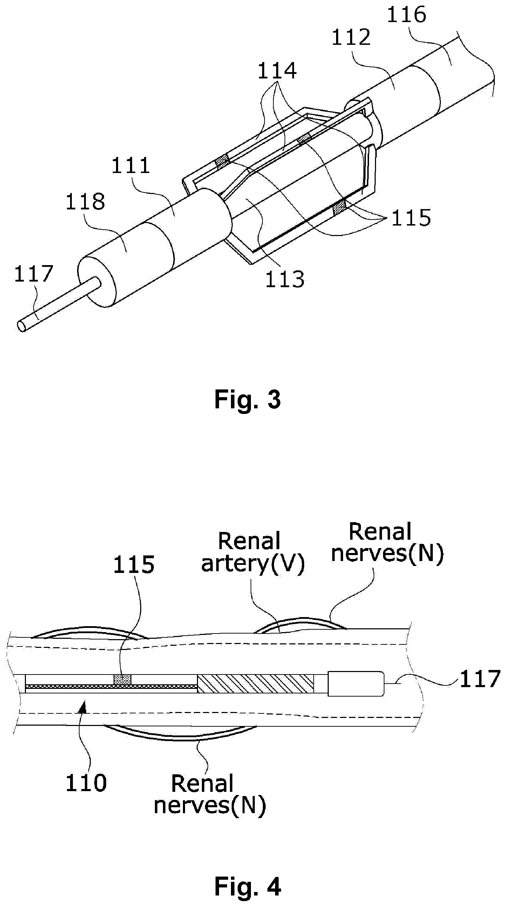

FIG. 3 is a perspective view schematically illustrating the configuration of the distal end side of the catheter according to an embodiment.

In this specification, the distal end of the catheter refers to an end on the side that reaches a treatment site among both ends in the longitudinal direction of the catheter. The distal end of the catheter is used interchangeably with distal end and distal part. In addition, the catheter has both ends extending long in one direction, and may be configured to move along the internal space of a blood vessel or the like.

Therefore, the proximal end of the catheter refers to the user's side among both longitudinal ends of the catheter. In this case, the proximal end of the catheter located on the operator's side is used interchangeably with 'proximal end' or 'proximal part'.

In other words, the end that is located on the opposite side of the proximal end and reaches the treatment region first at the forefront may be called the distal end. The distal end is used interchangeably with `distal end' or `distal part'.

In addition, the operator is located at the proximal end of the catheter and may adjust the movement of the distal end of the catheter. For example, the operator is located at the proximal end and may control the movement of the distal end of the catheter so that it is located in a desired target region in the body, and transfer the above-described energy to the electrode located at the distal end of the catheter at the target location. Thus, renal denervation may be performed. In addition, the distal end of the catheter is also referred to as a catheter head.

Referring to FIG. 3, the catheter may include a first frame 111, a second frame 112, a driving tube 113, a support member 114, the electrode 115, and a shaft body 116. Furthermore, the catheter may further include a guide wire 117.

The first frame 111 may be located at the distal end of the catheter. That is, the first frame 111 may be located at an end of both ends of the catheter that faces the treatment region or target location rather than the operator's side. In addition, the first frame 111 may have a hollow interior. This hollow may extend along the length of the catheter. Accordingly, the first frame 111 may have both ends open.

Furthermore, the hollow of the first frame 111 may extend parallel (horizontally) to the longitudinal direction of the catheter. Accordingly, the first frame 111 may have a structure in which each end facing in the longitudinal direction is open. That is, the first frame 111 may have openings disposed at each end facing in the longitudinal direction. By this configuration, certain components may be inserted or accommodated in the hollow of the first frame 111. In this specification, the hollow formed in the first frame 111 is referred to as a first hollow so as to be compared with the hollow formed in other components.

The second frame 112 may be located closer to the proximal end of the catheter than the first frame 111. That is, the second frame 112 may be located on the distal end of the catheter based on the entire portion of the catheter, but may be located on the proximal side of the catheter rather than the first frame 111. For example, the second frame 112 and first frame 111 may be located sequentially in the longitudinal direction of the catheter. However, the first frame 111 and second frame 112 may be spaced apart in the longitudinal direction, and the driving tube 113, support member 114, and electrode 115 to be described later may be located between the first frame 111 and the second frame 112.

In an embodiment, the second frame 112 may be spaced apart from the first frame 111 by a predetermined distance and may be located on the right side of the catheter, which may be considered the proximal side of the first frame 111.

Also, like the first frame 111, the second frame 112 may have a hollow interior formed therein. The hollow of the second frame 112 may extend along the longitudinal direction of the catheter. Accordingly, the second frame 112 may have openings located at both ends due to the hollow. Alternatively, the second frame 112 may be configured to have both ends open. Certain components may be inserted into the hollow of the second frame 112. In particular, the driving tube 113 may be inserted into the hollow of the second frame 112. In this specification, the hollow formed in the second frame 112 is referred to as a second hollow to be compared with the hollow formed in other components. The second hollow may also be configured with both ends open.

The first frame 111 and second frame 112 may be configured to be spaced a predetermined distance apart from each other along the longitudinal direction of the catheter. Also, a distance between the first frame 111 and the second frame 112 may be changed by the operator's manipulation. The configuration of the distance change between them will be described later.

In addition, the first frame 111 and/or second frame 112 may be made of various biocompatible materials. For example, these support members may be made of soft materials such as rubber or plastic, as well as hard materials such as metal. In particular, these support members are preferably made of soft and flexible materials. Furthermore, the first frame 111 is located at the front end of the catheter and is likely to come into contact with the inner wall of the blood vessel when the catheter moves along the blood vessel, etc.. Therefore, these support members are made of a soft and flexible material to prevent blood vessel damage, etc. and facilitate direction changes.

The driving tube 113 may be configured to extend long in one direction. Here, it may be said that the longitudinal direction of the driving tube 113 coincides with the longitudinal direction of the catheter. In particular, the driving tube 113 may extend from the distal end of the catheter to the proximal end of the catheter. Accordingly, the distal end of the driving tube 113 may be located on the side of the treatment region, and the proximal end of the driving tube 113 may be located on the operator's side. Therefore, the operator may move the driving tube 113 longitudinally by manipulating the proximal end of the driving tube 113.

The driving tube 113 may be formed to extend approximately in the left and right directions, and both the hollow left and right ends may be configured to be open. In this specification, the hollow of the driving tube 113 is referred to as a third hollow to distinguish it from the hollows of other components. In this way, the driving tube 113 may have a hollow interior, and since both ends of the hollow are configured to be open, certain components may be inserted or moved into the driving tube 113.

The driving tube 113 may be inserted into both the first hollow of the first frame 111 and the second hollow of the second frame 112. Also, the driving tube 113 may be fixed to the first frame 111 or second frame 112. That is, the driving tube 113 may be fixed to the first frame 111 or second frame 112 while being inserted into the hollows of the first frame 111 and second frame 112, respectively.

In addition, when the driving tube 113 is moved in the longitudinal direction, the first frame 111 and second frame 112 may be moved in the longitudinal direction.

For example, the driving tube 113 may be configured so that it is inserted into the hollow of the second frame 112 and freely moves in the longitudinal direction, that is, in the left and right directions in the drawing. Furthermore, when the driving tube 113 is moved in one direction, the first frame 111 coupled and fixed to the driving tube 113 may move in the same direction as the one direction. That is, when the operator pulls the proximal end of the driving tube 113, the driving tube 113 may move toward the proximal end of the catheter. In this case, the driving tube 113 may freely move in the hollow of the second frame 112 in the longitudinal direction, so the location of the second frame 112 may not change. In contrast, the first frame 111 is fixed to the driving tube 113, so when the driving tube 113 moves in the proximal direction (the one direction), the first frame 111 may move in the proximal direction, together with the driving tube 113.

Conversely, when the operator grabs and pushes the proximal end of the driving tube 113, the driving tube 113 may move in a direction opposite (the other direction) to the proximal direction, that is, toward the distal end of the catheter. This may cause the first frame 111 to move in the distal direction together with the driving tube 113. Accordingly, a separation distance between the first frame 111 and the second frame 112 may increase.

In this way, the separation distance between the first frame 111 and the second frame 112 may be adjusted depending on the moving direction of the driving tube 113. Correspondingly, the locations of the support member 114 and electrode 115 located between the first frame 111 and the second frame 112 and coupled to the first frame 111 and second frame 112 may also change.

The support member 114 may be configured to connect the first frame 111 and second frame 112 to each other while being between the first and second frames 111 and 112. That is, one end of the support member 114 may be connected and fixed to the first frame 111, and the other end of the support member 114 may be connected and fixed to the second frame 112. The support member 114 may be configured in the form of a rod or plate extending long in one direction.

When a distance between the first frame 111 and the second frame 112 narrows, the support member 114 may be configured to be bent at least in part as the distance between both ends becomes closer. As a result, the above-described bending region of the support member 114 may be moved away from the central axis of the catheter. That is, a portion of the bending region may be located outside the first frame 111 or second frame 112.

Also, when the first frame 111 and second frame 112 become close to each other and the support member 114 is bent, the bending region may move away from the central axis of the driving tube 113.

As described above, in the catheter according to the present invention, the distance between the first frame 111 and the second frame 112 may be adjusted by the driving tube 113. That is, as the driving tube 113 moves in the longitudinal direction, the distance between the first frame 111 and the second frame 112 may vary. Therefore, in the case of the catheter according to the present invention, the support member 114 may be bent or straightened through movement of the driving tube 113.

Meanwhile, the support member 114 must have a bending region formed according to the movement of the first frame 111 or second frame 112. Thus, the support member 114 may be made of a material that may be bent when the distance between both ends narrows. For example, the support member 114 may be made of a material such as metal or polymer. However, the present invention is not limited to a specific material of the support member 114, and the support member 114 may be made of various materials as long as a bending region may be formed in a portion of the support member 114.

A plurality of support members 114 may be provided between the first frame 111 and the second frame 112. For example, as illustrated in FIG. 3, three support members 114 may be provided. However, the present invention is not limited to a specific number of support members 114, and the support members 114 may be configured in various numbers. When a plurality of support members 114 are provided in this way, each support member 114 is bent at least in portion when the distance between the first frame 111 and the second frame 112 becomes closer. Therefore, the support member 114 may be configured so that the bending region moves away from the central axis of the driving tube 113.

The electrode 115 is mounted on the support member 114 and may generate heat by receiving power. Also, in this way, the heat generated by the electrode 115 may apply thermal stimulation to surrounding tissues. For example, the heat generated by the electrode 115 may ablate the surrounding tissue. In this case, the electrode 115 may generate heat of approximately 40 to 80 °C to ablate the nerves around the blood vessels, thereby blocking the nerves. However, it is apparent that the temperature of the heat generated by the electrode 115 may be implemented in various ways depending on the use or purpose of the catheter.

Since the electrode 115 may apply heat to the nerve tissue located around the blood vessel by contacting the blood vessel wall, it is preferable that the electrode 115 may be in close contact with the blood vessel wall. Accordingly, the electrode 115 may have a curved outer surface so that the surface of the electrode in contact with the inner wall of the blood vessel may correspond to the shape of the inner wall. For example, the electrode 115 may have a circular, semicircular, or oval cross-sectional shape.

The electrode 115 may be provided in the bending region of the support member 114. The bending region of the support member 114 may be configured to be furthest from the central axis of the catheter according to a change in the distance between the first frame 111 and the second frame 112. Therefore, when the electrode 115 is provided in the bending region of the support member 114, the electrode 115 may be located closer to the inner wall of the blood vessel than other components (support member, first and second support members, etc.)..

In this configuration, when the degree of bending of the support member 114 becomes the most severe as the distance between the first frame 111 and the second frame 112 becomes shorter, the electrode 115 may approach the inner wall of the blood vessel. In this case, it may be said that the catheter head is open. Conversely, when the support member 114 is stretched or the degree of bending is weakened as the distance between the first frame 111 and the second frame 112 increases, the electrode 115 may be furthest from the inner wall of the blood vessel. In this case, it may be said that the catheter head is closed.

The electrode 115 may generate heat or transmit high-frequency energy using radio frequency (RF). For example, the electrode 115 may be electrically connected to a high-frequency generating unit and radiate the high-frequency energy to perform renal denervation.

The shaft body 116 may extend long in one direction. For example, the shaft body 116 may extend in the longitudinal direction of the catheter.

In addition, the shaft body 116 may extend approximately in the left and right directions, as illustrated in FIG. 3 . In particular, the shaft body 116 may extend long from the distal end of the catheter where the first frame 111 and second frame 112 are located to the proximal end of the catheter where the operator is located.

As described above, the catheter 110 according to the present invention may further include a guide wire 117.

The guide wire 117 is a wire for guiding the catheter 110 to the treatment site, and may be configured to reach the treatment site before the catheter. This guide wire 117 may extend in one direction or in a longitudinal direction. Further, the guide wire 117 may be inserted into the hollow of the driving tube 113. In this configuration, the guide wire 117 moves along the inside of the blood vessel to reach the treatment site first, and the driving tube 113, first frame 111, second frame 112, support member 114, etc. may be guided to the treatment site with the guide wire 117 inserted into the hollow. Furthermore, there may be an ultrasonic sensor in the guide wire 117 that functions as a guide and at the same time as a detection means to be described later.

In addition, an end tip 118 may be further included at the distal end of the catheter, that is, in front of the distal end of the catheter head.

The end tip 118 is configured in the form of a hollow tube and may be made of a soft and flexible material. In particular, the end tip 118 may be formed of a composition containing polyether block amide (PEBA).

According to this configuration of the present invention, when the distal end of the catheter moves along the blood vessel, etc., the end tip 118 made of a soft and flexible material is located at the front, thereby reducing damage to the blood vessel, etc. and facilitating direction changes. Moreover, in the case of the end tip 118 made of the above material, X-ray imaging may be conducted, so it may be easy to determine the location of the catheter head.

FIG. 4 is a diagram illustrating a portion of FIG. 2.

Referring to FIG. 4 (hereinafter in addition refer to FIGS. 1 and 3), the catheter 110 may further include the guide wire 117 disposed at the distal end 110a. Accordingly, the distal end 110a of the catheter 110 may be easily moved to the target location by the guide wire 117.

For example, the catheter 110 and guide wire 117 move to the target within the blood vessel V according to the user's manipulation, and renal denervation may be performed through the electrode. In addition, evaluation of renal denervation outcomes may be performed. Further, for the evaluation of renal denervation, a detection means such as, for example, an ultrasonic sensor, an impedance sensor, and an optical sensor may be attached to the guide wire 117. The detection means may be disposed on the catheter, and will be described below based on its deposition at the distal end of the catheter. A detailed explanation of this will be provided later.

In addition, a plurality of electrodes 115 are disposed at the distal end of the catheter 110 without the above-described guide wire, and renal denervation and evaluation of renal denervation outcomes may be performed through the plurality of electrodes 115.

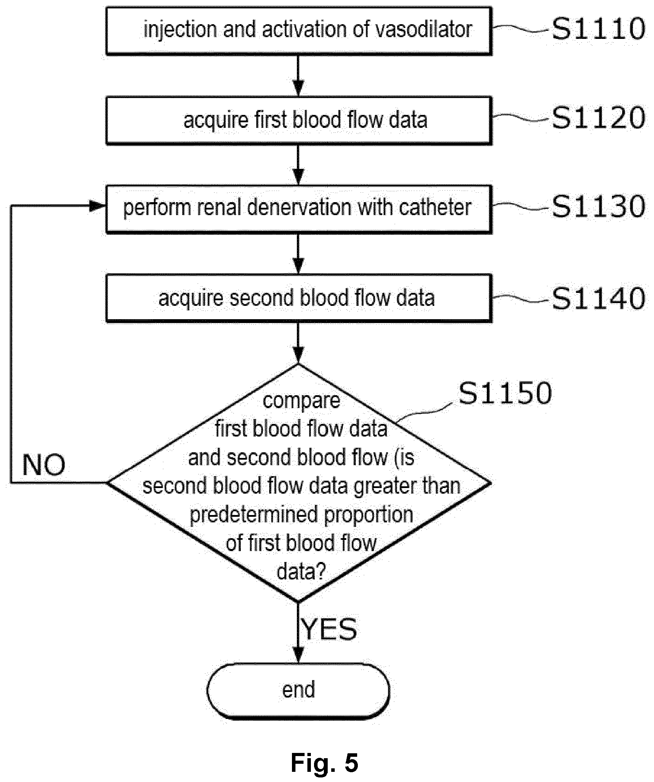

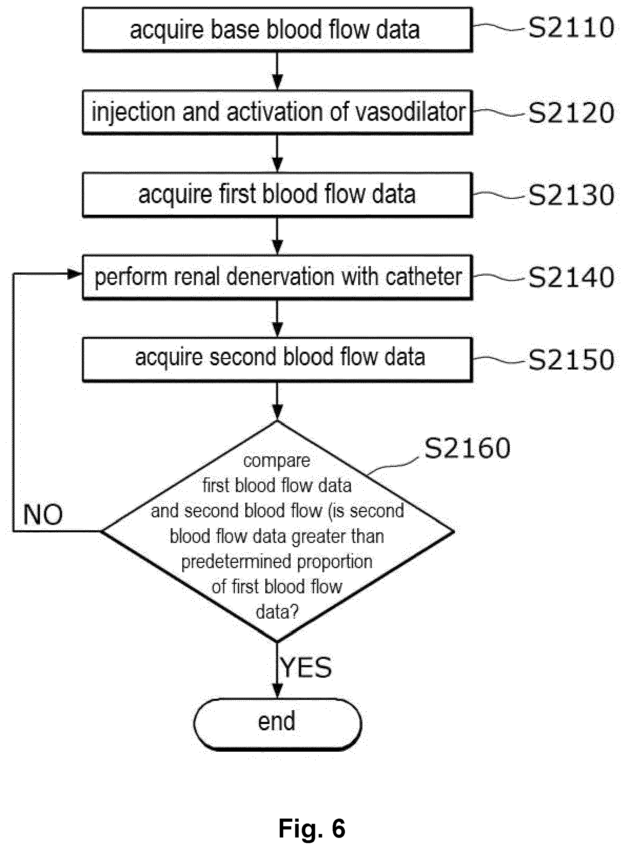

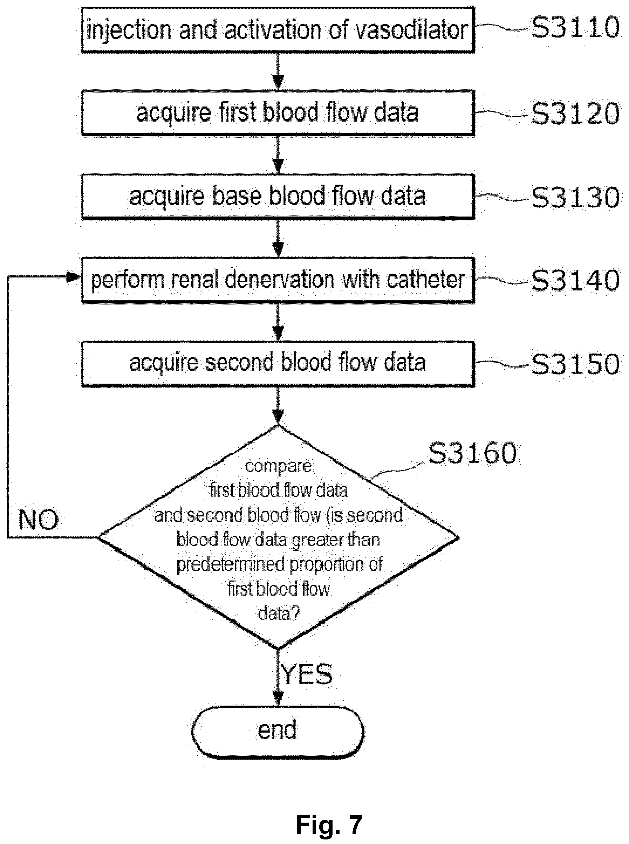

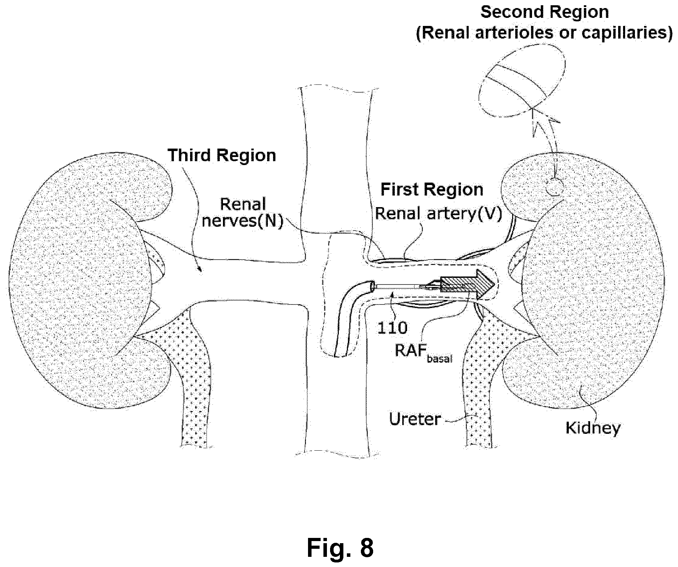

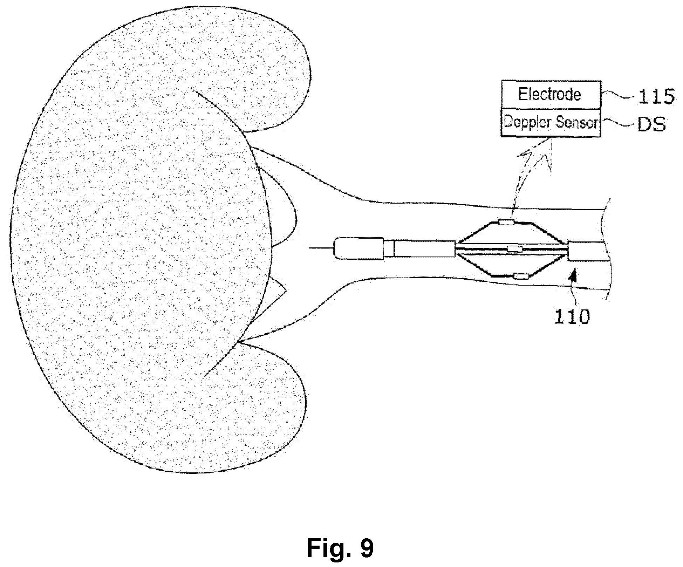

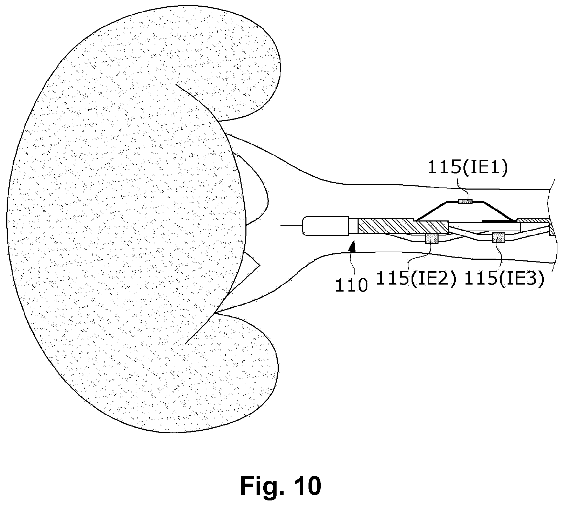

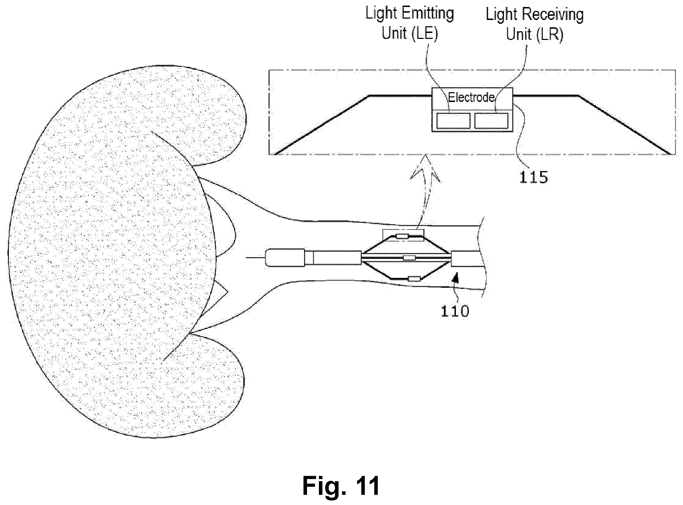

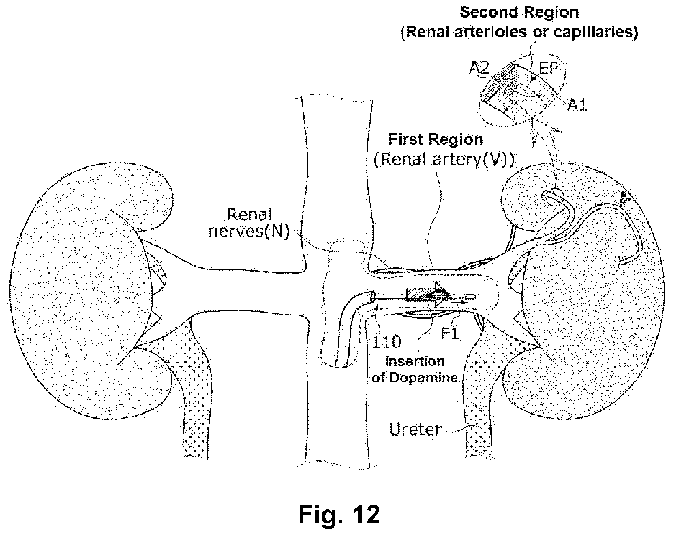

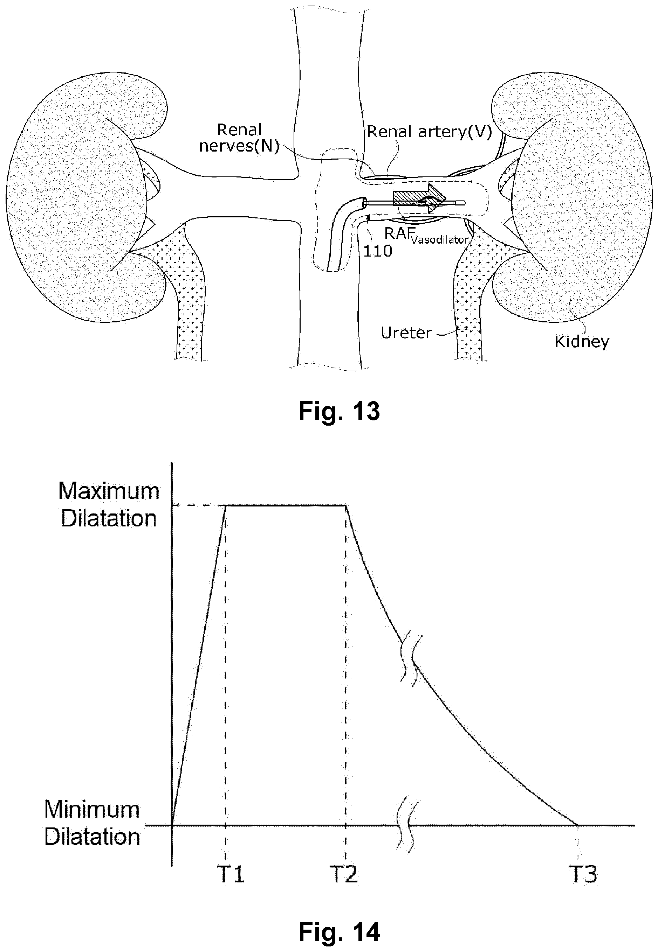

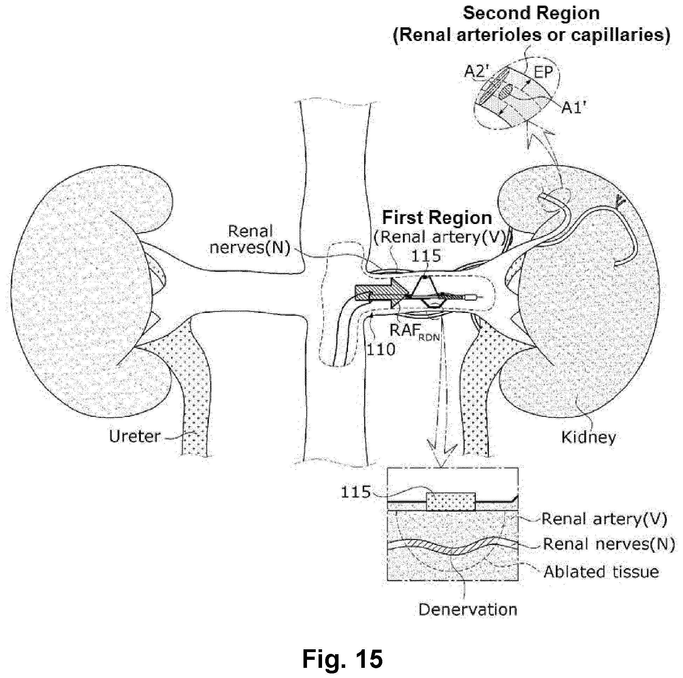

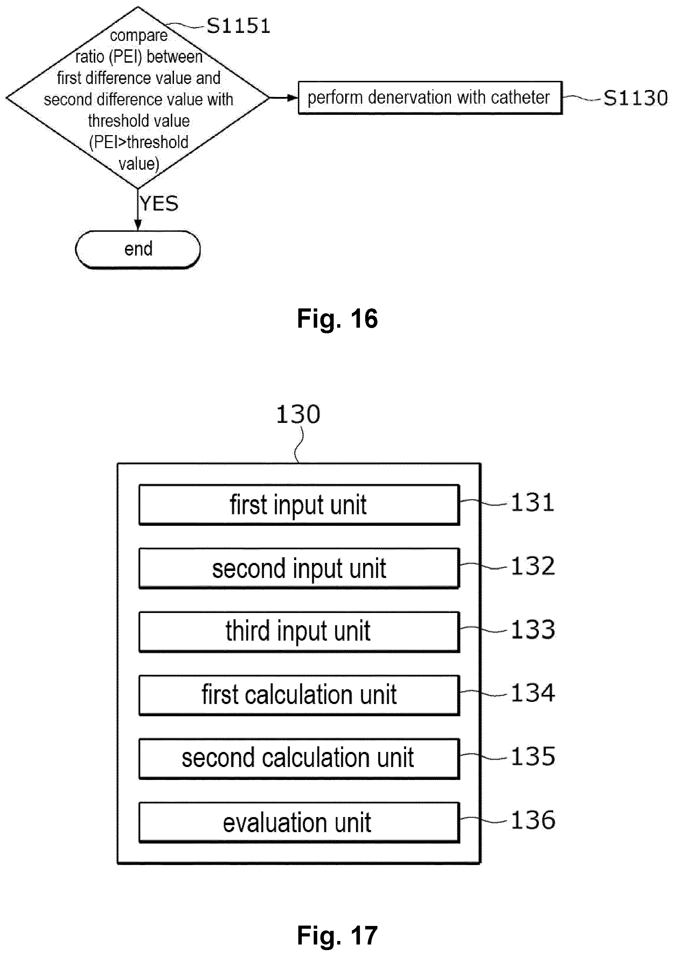

FIG. 5 is a flowchart of a method for evaluating an outcome of renal denervation according to an embodiment, FIG. 6 is a flowchart of a method for evaluating an outcome of renal denervation according to another embodiment, FIG. 7 is a flowchart of a method for evaluating an outcome of renal denervation according to another embodiment, FIG. 8 is a diagram explaining a method of acquiring base blood flow data in a method for evaluating an outcome of renal denervation according to the present invention. FIG. 9 is a diagram explaining an example of a method of acquiring blood flow data, FIG. 10 is a diagram explaining another example of a method of acquiring blood flow data, FIG. 11 is a diagram explaining another example of a method of acquiring blood flow data, FIG. 12 is a diagram explaining injection of a vasodilator in a method for evaluating an outcome of renal denervation according to an embodiment, FIG. 13 is a diagram explaining a method of acquiring first blood flow data in a method for evaluating an outcome of renal denervation according to an embodiment, FIG. 14 is a graph of an action of a vasodilator in a method for evaluating an outcome of renal denervation according to an embodiment, FIG. 15 is a diagram explaining renal denervation using a catheter and acquiring second blood flow data in a method for evaluating an outcome of renal denervation according to an embodiment, FIG. 16 is a flowchart explaining comparison of first blood flow data and second blood flow data in a method for evaluating an outcome of renal denervation according to an embodiment.

Referring to FIG. 5, a method for evaluating an outcome of renal denervation according to an embodiment may comprise the steps of acquiring first blood flow data from a first region in a renal blood vessel after a vasodilator injected into the first region is activated in a second region in the renal blood vessel, and acquiring second blood flow data from the first region after renal denervation is performed with a catheter for denervation. The renal blood vessels may include renal arteries, renal arterioles, renal capillaries, and renal veins. The first region may be the renal artery. The second region may be a renal arteriole or renal capillary.

More specifically, the method for evaluating an outcome of renal denervation may include the steps of injecting the vasodilator and activating the vasodilator in the second region (renal arteriole) (S1110), acquiring the first blood flow data from the first region (renal artery) (S1120), performing the renal denervation with the catheter located in the first region (renal artery) (S1130), acquiring the second blood flow data from the first region (renal artery) after performing the renal denervation (S1140), and comparing the first blood flow data and the second blood flow data (S1150).

First, in the step (S 1110) of injecting the vasodilator and activating vasodilator, the injection of the vasodilator may be performed in a specific region of the first region (renal artery) within the renal blood vessel. The vasodilators (e.g., Dopamine, Dobutamine, adenosine, prostacyclin, nitric oxide, etc.) are injected into the renal vasculature, but the injection location of the vasodilator is determined so that the direct effect on the renal artery is minimized and the vasodilator is activated in the renal arterioles connected to the renal artery. Therefore, by acquiring data on blood flow in the renal artery while the renal arterioles are maximally vasodilated, accurate evaluation of renal denervation may be performed.

That is, the vasodilator is injected into a specific location of the renal artery (i.e., first region) to minimize the time the vasodilator stays in the renal artery. In doing so, the evaluation for the renal denervation is performed using the blood flow data acquired through the activated and dilated renal arterioles primarily in the renal arterioles (i.e., region 2) without significantly affecting the diameter or size of the renal artery vessels.

In this case, when the vasodilator is injected into a specific location of the renal artery, the renal arterioles may be dilated to the maximum blood vessel level without affecting the vasodilation of the renal artery. Further, in order to accurately measure the blood flow in the renal artery, the blood flow must be measured in the activated state. In other words, the blood flow in the renal artery must be measured while minimizing the effect of the vasodilator on the renal artery and maximally dilating the blood vessels in the renal arterioles in order to accurately measure the maximum blood flow unique to each patient.

Accordingly, it is possible to acquire blood flow data in the same state as when the sympathetic nerve was ablated from the renal artery.

Therefore, the evaluation and determination on the renal denervation may be performed quickly and accurately by comparing the maximum blood flow data in the renal artery unique to each patient (first blood flow data) with the blood flow data in the renal artery obtained after the renal denervation by the catheter (second blood flow data), minimizing the burden on patients.

In addition, based on the comparison results between the blood flow data (1 st and 2nd blood flow data), if the comparison results are greater than or equal to a predetermined threshold, it may be determined that the procedure has been successful, and if the comparison results are lower than or equal to a predetermined threshold, it is a result of the procedure not being performed properly, so it may be determined whether to perform the procedure again.

The vasodilators may include any one of dopamine, dobutamine, adenosine, prostacyclin, nitric oxide, bradykinin, papaverine, dipyridamolum, diuretin, theophylline, minoxidil, and isosorbide.

In contrast, when the dilating agents such as nitroglycerin are used, blood vessels such as the renal artery and renal arteriole are dilated. Accordingly, it may become difficult to acquire accurate blood flow data in the case of nitroglycerin. As a result, it may be difficult to acquire blood flow information, such as the same blood flow volume, as when the renal nerve is ablated from the renal artery through injection of nitroglycerin.

As another embodiment, when nitroglycerin is injected during a procedure, vasodilation (activation) by nitroglycerin must be maintained when first blood flow data, second blood flow data, and base blood flow data are acquired. Therefore, if activation by nitroglycerin decreases before blood flow data acquisition, nitroglycerin must be reinjected. This is because the first blood flow data, second blood flow data, and base blood flow data must be acquired while the variable factors caused by nitroglycerin are the same.

The vasodilator in the renal denervation according to an embodiment is different from the above-described nitroglycerin in terms of the effect upon activation and the location of activation. Furthermore, since the vascular tonic agent according to an embodiment has a shorter duration compared to nitroglycerin, evaluation and determination on the renal denervation may be performed quickly, minimizing the burden on the patient.

The method and device for evaluating an outcome of renal denervation according to an embodiment may easily acquire the patient's own maximum blood flow as blood flow data without vasodilation of the renal artery itself, as described above. Therefore, the vasodilators (e.g., dopamine, adenosine, etc.) proposed in the present invention have a short duration, minimizing the time to acquire blood flow data in a state that is not affected by activation of the vasodilators. As a result, the procedure time for patients undergoing renal denervation is reduced, and the risk of the procedure may be also reduced.

In addition, by injecting the vasodilator into the renal vasculature before renal denervation, data on blood flow within the renal artery in the state of maximum vasodilation may be acquired. In other words, the method and device for evaluating an outcome of renal denervation according to the present invention may acquire blood flow data that is not affected by the compound factors of blood vessels that occur after renal denervation. For example, compound factors in blood vessels may include muscle cramps (spasm) due to stimulation of the ache nerve after renal denervation, changes in blood pressure due to stimulation of the sympathetic nerve, etc. In other words, if data on blood flow in the state of maximum vasodilation is acquired by injecting the vasodilator into the renal blood vessels after renal denervation, the outcomes will be different from those before the renal denervation, making it impossible to acquire accurate comparison value data.

In addition, according to an embodiment, the vasodilator may be locally injected into the renal artery to minimize the time it stays in the renal artery, thereby dilating the renal arterioles to the maximum blood vessel level without affecting vasodilation of the renal arteries. Accordingly, the method and device for evaluating an outcome of renal denervation according to the present invention may acquire blood flow data in the same state as the state in which the sympathetic nerve is ablated from the renal artery.

Referring to FIG. 12 together, the vasodilator may be injected into the renal artery through the catheter or other member as described above (S1110). Specifically, when dopamine, which is the vasodilator, is injected into the renal artery, it may block sympathetic nerve signals to the renal small arteriole in the kidney, which is thinner (e.g., smaller in diameter or thickness) than the renal artery. Accordingly, the vasodilation of the renal arterioles may occur (EP), as described above. For example, the diameter or cross-sectional region A1 of the renal arteriole before injection of the vasodilator may be smaller than the diameter or cross-sectional region A2 of the renal arteriole after injection of the vasodilator. With this configuration, the blood flow to the renal arterioles increases, ultimately increasing the blood flow rate F1 in the renal arteries (e.g., blood vessels, V). This may correspond to a transient hyperemic state or transient renal denervation state and may be maintained for a predetermined period of time of 4 to 6 minutes (preferably within 5 minutes).

Referring to FIG. 13 together, first blood flow data in the first region (renal artery) may be acquired (S 1 120). In other words, blood flow in the renal artery may be increased by the vasodilators corresponding to the state of complete renal denervation (the same state as the state in which the sympathetic nerve is ablated in the renal artery). For example, blood flow may be maximum in the renal artery. In this case, the first blood flow data corresponds to blood flow data in the renal artery within a predetermined time after injection of the vasodilator, hereinafter referred to as "RAF_{Vasodilator}". This first blood flow data may be acquired within the predetermined time period described above. For example, it may be the maximum or average value among the blood flow velocities acquired within a predetermined time.

In this way, by injecting the vasodilator locally into the renal artery rather than by intravascular injection, the renal arterioles are maximally dilated, thereby acquiring more accurate first blood flow data as data corresponding to the state in which renal denervation is performed without changing the diameter of the renal artery.

Referring to FIG. 14, the diameter of the renal arteriole increases during a first time T1 due to the vasodilator, and then the diameter of the renal arteriole is maintained at the maximum from the first time T1 to a second time T2 (maximum dilatation). Further, at the second time T2 or the third time T3, the vasodilator such as dopamine is washed away, and the diameter of the renal arteriole finally returns to the state (0) before the vasodilator is injected. For example, the third time T3 may be within 30 minutes. However, this is a graph that removes other compounds that cause the vasodilation of renal arterioles.

In an embodiment, the vasodilator may be injected at 50 µg/kg or less, and the second time T2 may vary depending on the amount, but may be 5 minutes (min) or less in consideration of half-life, etc. For example, the vasodilator may have a half-life of 1 to 2 minutes. In addition, the first time T1 may be smaller than a difference between the second time T2 and the first time T1. Accordingly, first blood flow data may be acquired between 10 seconds and 5 minutes after injecting the vasodilator into the renal artery. In addition, renal nerve denervation may be performed 5 minutes after injection of the vasodilator. If the procedure is performed outside of this, there is a problem of acquiring inaccurate first blood flow data. However, there is room for change depending on the amount of vasodilator injected, etc.

In this specification, the blood flow data may be acquired by an acquisition means from the blood flow information calculated by peak-to-peak, average, cross-sectional region comparison, blood flow, peak blood flow velocity, blood flow resistance, etc. of the signals received by the detection means. More specifically, the blood flow information includes blood flow volume, blood flow speed, blood flow resistance, etc., and the acquisition means may acquire or receive any one of the blood flow information from the detection means. For example, the first blood flow data, second blood flow data, and base blood flow data acquired by the acquisition means may be the same category of blood flow information, for example, blood flow volume. For example, in the case of an impedance sensor, when the blood flow speed increases, the impedance may increase, and when the blood flow speed decreases, the impedance may decrease. Using this, the blood flow rate may be calculated as the impedance or peak-to-peak or average value per cycle of the impedance value. Furthermore, in case of the ultrasonic sensor, there may be a signal graph opposite to that of an impedance sensor.

The detection means may be located within the renal artery. It may also be located within a region between the aorta and kidney.

Further, the renal denervation may be performed with the catheter located in the first region (renal artery) (S1130). The renal denervation may be performed by means of the above-described electrodes on the catheter. In this case, the electrode of the catheter is in contact with the renal nerve N located in contact with or adjacent to the blood vessel V, and the renal nerve N may be ablated by transmitting heat, etc. to the renal nerve N. The vasodilator is characterized by a sufficiently short half-life so as not to affect the step of performing the renal denervation.

The renal denervation may be performed multiple times and may be performed multiple times at various locations within the renal artery. For example, the number of branches of the renal artery may vary depending on the patient, and the renal denervation may be performed multiple times depending on the number of branches of the renal artery.

Referring to FIG. 15 together, the second blood flow data within the first region (renal artery) may be acquired after performing the renal denervation (S1140). If the renal denervation is performed, the vasodilatation (EP') of the renal arterioles may be permanently increased, as in the case of dopamine infusion. That is, after the renal denervation, the vessel diameter or cross-sectional region of the renal arteriole increases compared to the vessel diameter or cross-sectional region of the renal arteriole before the renal denervation (A1'->A2'). Here, 'A1" represents the vessel diameter or cross-sectional region of the renal arteriole before the renal denervation, and 'A2" represents the diameter or cross-sectional region of the renal arteriole after the renal denervation.

Further, the first blood flow data and second blood flow data may be compared (S1150). For example, the renal nerves N may be disposed outside the renal artery V. In this case, part of the tissue is ablated by the energy transferred by the electrode 115, and renal nerves adjacent to the ablated tissue may also be ablated. In an embodiment, if the second blood flow data is lower than a predetermined ratio of the first blood flow data, the renal denervation may be performed again. Alternatively, the renal denervation may be performed again when the ratio of the second blood flow data and the first blood flow data is lower than a predetermined threshold. Further, after re-performing the nerve resection, the first blood flow data and second blood flow data may be compared again. In this case, a predetermined ratio may be set according to the patient's condition. Further, the second blood flow data is blood flow data in the renal artery after the renal denervation and corresponds to "RAF_{RDN}" hereinafter.

Through this, when the renal denervation of the kidney is completely (e.g., 100%) achieved, vasodilation of all renal arterioles is permanently achieved, and the blood flow rate in the renal arteries may be maximized according to vasodilation of renal arterioles. That is, the second blood flow data may be similar to the first blood flow data.

However, when the renal denervation is incomplete (e.g., lower than 80%), partial vasodilation of the renal arterioles occurs, and the flow rate within the renal artery may be slower compared to a case where the renal denervation is complete. That is, the second blood flow data may be lower than or equal to a predetermined ratio of the first blood flow data. In this case, the second blood flow data may be acquired again by returning to the above-described step (S1130) and re-performing the renal denervation in the renal artery. In addition, when the second blood flow data has a predetermined ratio or higher value of the first blood flow data, renal denervation through the catheter may be completed and the catheter may be separated from the patient's body. In this way, by acquiring the second blood flow data after acquiring the first blood flow data, evaluation of renal denervation may be performed even if the first blood flow data is performed once regardless of the number of renal denervation. In other words, since there is no need to repeatedly acquire the first blood flow data, which is the standard for evaluating renal denervation, the procedure time may be reduced and it may prevent complex fluctuation compounds in blood vessels caused by the renal denervation.

In addition, in the method for evaluating an outcome of renal denervation according to an embodiment, the first blood flow data and second blood flow data are compared as described above, and then the outcome of the renal denervation may be evaluated. For example, if the comparison result is lower than a predetermined threshold, an alarm may be provided to re-perform the renal denervation. Here, the threshold may be, for example, 80% as the threshold for the ratio of the second blood flow data to the first blood flow data (second blood flow data/first blood flow data × 100). Then, the step of acquiring the second blood flow data may be repeated again after re-performing the renal denervation. Furthermore, in the method for evaluating an outcome of renal denervation results according to an embodiment, before injecting the vasodilator or performing renal denervation with the catheter located in the renal artery (or before acquiring the second blood flow data), base blood flow data may be acquired. Each step will be described in embodiments below.

Referring to FIG. 6, a method for evaluating an outcome of renal denervation according to another embodiment may comprise the steps of acquiring base blood flow data from a first region in renal nerve (renal artery), acquiring first blood flow data from the first region after a vasodilator injected into the first region is activated in a second region in the renal blood vessel, and acquiring second blood flow data from the first region after renal denervation is performed with a renal denervation catheter.

More specifically, the method for evaluating an outcome of renal denervation may comprise the steps of acquiring base blood flow data from a first region (renal artery) (S2110), injecting a vasodilator and activating the vasodilator (S2120), acquiring first blood flow data in the renal artery after the vasodilator is activated (S2130), performing renal denervation with a catheter located in the artery (S2140), acquiring second blood flow in the renal artery after renal denervation is performed (S2150), and comparing the first blood flow data and the second blood flow data (S2160).

The step of injecting a vasodilator and activating the vasodilator (S2120) may be performed in the same way as the step of injecting a vasodilator and activating the vasodilator (S1110) described above in FIG. 5, the step of acquiring first blood flow data in the renal artery (S2130) may be performed in the same way as the step of acquiring the first blood flow data in the renal artery (S 1 120) described above in FIG. 5, the step of performing renal denervation with a catheter located in the renal artery. (S2140) may be performed in the same way as the step of performing renal denervation with a catheter located in the renal artery (S1130) described above in FIG. 5, and the step of acquiring second blood flow in the renal artery after performing the renal denervation (S2150) may be performed in the same way as the step of acquiring second blood flow data in the renal artery after performing the renal denervation (S1140) described above in FIG. 5. Further, in the method for evaluating an outcome of renal denervation according to an embodiment, the step of acquiring base blood flow data (S2110) may be performed before the vasodilator is injected.

Referring to FIGS. 8 and 16 together, the base blood flow data may be acquired within the first region (renal artery) before the vasodilator (e.g., dopamine) is injected. In an embodiment, the base blood flow data is blood flow data in the renal artery before the vasodilator is injected, and may correspond to "RAF_{basal}" hereinafter.

In this case, in the step of comparing the first blood flow data and the second blood flow data (S2160), referring to FIG. 16, a ratio (e.g., corresponding to 'PEI') between a first difference value and a second difference value may be compared with a threshold value (S1151).

In an embodiment, the method for evaluation an outcome of renal denervation may further comprise calculating a first difference value representing a difference value between the first blood flow data and the base blood flow data before the step of comparing the first difference value and the second difference value, and calculating a second difference value representing a difference between the second blood flow data and the base blood flow data. In this case, the calculation order of the first difference value and second difference value may be simultaneously or reversed.

Further, the renal denervation outcome may be evaluated according to the result of comparing the first difference value and the second difference value. For example, the renal denervation outcome may be evaluated through a ratio or proportion between the first difference value and the second difference value. Here, as described above, the first difference value means a difference value between the first blood flow data and the base blood flow data, and the second difference value means a difference value between the second blood flow data and the base blood flow data. Further, the ratio between the first difference value and the second difference value may mean a ratio of the second difference value to the first difference value or a ratio of the first difference value to the second difference value. Hereinafter, the description will be based on the ratio of the second difference value to the first difference value.

Furthermore, if the ratio between the first difference value and the second difference value is lower than a predetermined threshold value, an alarm may be provided to re-perform the renal denervation. Here, the threshold value may be, for example, a threshold value of the ratio of the second difference value to the first difference value. Also, after re-performing the renal denervation, the second difference value may be calculated again and the comparison of the first difference value and the second difference value may be repeated. In addition, the first blood flow data, second blood flow data, and base blood flow data may be acquired while the driving tube is moved distally or proximally as described above. For example, the first blood flow data, second blood flow data, and base blood flow data may be acquired while the driving tube is moved to either the distal or proximal side. The first blood flow data, second blood flow data, and base blood flow data are acquired while the catheter in the renal artery is in the same state, so that errors in each blood flow data may be minimized.

In addition, the first blood flow data, second blood flow data, and base blood flow data may be acquired by the blood flow information detected in the first region within the renal artery by the above-described detection means. For example, the first blood flow data, second blood flow data, and base blood flow data may be acquired while the position of the driving tube is the same. In other words, the operator manipulates the proximal end of the driving tube to move the driving tube longitudinally, while manipulating the proximal end of the driving tube the same amount, so that the first blood flow data, second blood flow data, and base blood flow data may be acquired while the driving tube is moved by the same amount in the longitudinal direction. As a result, when the catheter is present at the same position within the renal blood vessel, especially within the first region, each of the first blood flow data, second blood flow data, and base blood flow data is acquired, thereby minimizing the influence on the shape, structure, etc. of the renal blood vessel of each patient. In other words, an accurate assessment of renal denervation may be performed.

In addition, in an embodiment, if the ratio between the first difference value and the second difference value is greater than a threshold value, it is determined that the renal nerve has been ablated beyond a target value, and the catheter may be separated from the patient's body. On the other hand, if the ratio (PEI) between the first difference value and the second difference value is lower than a threshold value, the renal denervation above the target value has not been achieved, and the renal denervation using the above-described catheter is re-performed (S1130), The second blood flow data may be acquired again (S2150). Then, the re-acquired second blood flow data and the previously acquired first blood flow data may be re-compared, or the ratio between the first difference value and the second difference value may be compared with a threshold value as described above. Accordingly, as described above, the first blood flow data and base blood flow data only need to be measured once. In other words, there is no need to repeatedly acquire the first blood flow data and base blood flow data, which are references for evaluating renal denervation, and thus the procedure time may be reduced.

In this case, the ratio PEI between the first difference value and the second difference value satisfies Equation 1. The first and second difference values are the difference values between the first blood flow data RAF_{Vasodilator} and the base blood flow data RAF_{basal} and between the second blood flow data RAF_{RDN} and the base blood flow data RAF_{basal}, respectively. The ratio between the first difference value and the second difference value (procedural endpoint index, PEI) = )=(RAFRDN - RAFbasal)/(RAFVasodilator - RAFbasal)

Here, RAF_{RDN} is renal artery blood flow data after performing the renal denervation, which corresponds to the second blood flow data, RAF_{basal} is basic blood flow data for blood flow in the renal artery, which corresponds to the base blood flow data, and RAF_{Vasodilator} is blood flow data in the renal artery after injection of the above vasodilator, which corresponds to the first blood flow data. Such blood flow data may include blood flow speed, blood flow volume, blood flow resistance, etc. Specifically, the first blood flow data, second blood flow data, and base blood flow data described later, which are blood data, may be any one of blood flow information, such as blood flow, blood flow rate, which is the amount of blood flow per unit of time, peak speed, and blood flow resistance. Also, the first blood flow data, second blood flow data, and base blood flow data described later may belong to the same category in blood flow information. For example, the first blood flow data, second blood flow data, and base blood flow data described later may all be blood flow. Alternatively, the first blood flow data, second blood flow data, and base blood flow data described later may be blood flow speeds. Based on this, the following description will be provided. Furthermore, hereinafter, a threshold value (e.g., first threshold value, second threshold value) may vary depending on the category of blood flow information. For example, a threshold value for a ratio between the first difference value and the second difference value may be 0.8 for blood flow volume, but may be different from or equal to 0.8 for blood flow velocity.

Also, when a ratio between the first difference value and the second difference value is equal to or greater than a threshold value, renal denervation through the catheter may be terminated and the catheter may be separated from the patient's body. Alternatively, if a ratio between the first difference value and the second difference value is lower than a threshold value, renal denervation through the catheter may be performed again as described above.

In addition, in this embodiment, the first blood flow data RAF_{Vasodilator}, second blood flow data RAF_{RDN}, and base blood flow data RAF_{basal} may be acquired from the blood flow information detected by the detection means, for example, an ultrasonic sensor, an impedance sensor, or an optical sensor.

As described above, the ultrasonic sensor, impedance sensor, and optical sensor may be located on the catheter, guide wire, etc. In an embodiment, the ultrasonic sensor may include a Doppler sensor. Furthermore, a Doppler sensor disposed on the above-described guide wire may be used as the detection means. In addition, the impedance sensor may use the catheter electrode as is or may be located adjacent to the electrode of the catheter to acquire the blood flow data. For example, the impedance value may be detected by a catheter electrode, or the impedance value may be detected by a separate impedance sensor.

In this way, by acquiring the base blood flow data RAF_{basal} in the method and device for evaluating an outcome of renal denervation according to the embodiment, it is possible to accurately determine whether renal denervation has been completely conducted for each patient.

For example, the base blood flow data RAF_{basal} may be different for each patient. In other words, patients with high blood pressure may have lower base blood flow data RAF_{basal} than the general population. In other words, compared to the general population, hypertensive patients may have more capillary constriction caused by the sympathetic nerve, and hypertensive patients may experience greater changes in blood flow data due to renal denervation based on base blood flow data (RAF_{basal}), compared to the general population. Conversely, the amount of change between the first blood flow data and the second blood flow data (hereinafter referred to as 'blood flow change amount') in a patient with high blood flow (normal times) may be smaller than the amount of change in blood flow in a patient with high blood flow (normal times). In this case, the relative blood flow change due to renal denervation according to an embodiment may be easily calculated by using the base blood flow data RAF_{basal}. In other words, the method and device for evaluating an amount of renal denervation according to an embodiment may accurately evaluate renal denervation by using the base blood flow data RAF_{basal} even if the physical condition of each patient is different.

In addition, in the various embodiments above, the step of acquiring the second blood flow data may be performed a predetermined time after performing the renal denervation. Even if contraction of the renal artery occurs due to muscle stiffness due to pain during the renal denervation, recovery time for the renal artery is given after a predetermined period of time, and then the second blood flow data is acquired, so that the second blood flow data may be acquired after complex compounds are removed. Therefore, more accurate blood flow data may be acquired.

Also, the first blood flow data RAF_{Vasodilator} and base blood flow data RAF_{basal} may be acquired before the renal denervation. As a result, it is possible to avoid being influenced by complex compounds occurring in the renal artery after the renal denervation.

In addition, in another embodiment, the step of acquiring the second blood flow data may be performed by performing renal denervation of the contralateral renal artery (third region) and then returning to the initial renal artery (first region). Even if constriction of the renal artery occurs due to muscle stiffness due to pain during the renal denervation, after performing the renal denervation on the contralateral renal artery (third region), and then allowing some recovery time for the renal artery, and then returning to the first region, the second blood flow data is acquired. Thus, the second blood flow data may be acquired after the complex compounds are removed. Therefore, more accurate blood flow data may be acquired. Further, the step of acquiring the second blood flow data may include acquiring a second-1 blood flow data within the renal artery (first region) and then sequentially acquiring a second-2 blood flow data within the other renal artery (third region).

For example, after the renal nerve is ablated in either the right renal artery or the left renal artery (first region), the catheter is moved to the opposite renal artery (third region) to perform ablation and then returned to the original location of the renal artery (first region) to sequentially acquire the second blood flow data (RAF_{RDN}) from one artery and the other artery. Here, the second blood flow data acquired from one artery may correspond to the second-1 blood flow data, and the second blood flow data acquired from the other artery may correspond to the second-2 blood flow data.

In addition, in the various embodiments described above, the second blood flow data is acquired after the vasodilator is injected and the first blood flow data is acquired, so even when renal denervation is performed multiple times, the acquisition of the first blood flow data following injection of the vasodilator may be performed once. In other words, by not acquiring the first blood flow data multiple times, the procedure time may be reduced and changes in the first blood flow data caused by various compounds may be eliminated.

Referring to FIG. 9, the blood flow data (e.g., first blood flow data RAF_{Vasodilator}, second blood flow data RAF_{RDN} and base blood flow data RAF_{basal}) within the renal artery may be acquired through the ultrasonic sensor (DS) disposed adjacent to the end of the guide wire or electrode 115 of the catheter. For example, the ultrasonic sensor may be a Doppler sensor, may be plural, and may be spaced apart along the flow of blood. Accordingly, each of the plurality of ultrasonic sensors detects the blood flow rate in the proximal part of the renal artery, the blood flow rate in the distal part of the renal artery (renal arteriole or a position adjacent to the kidney), and the blood flow between the proximal part and the distal part (e.g., the middle part), thereby improving the accuracy of the blood flow rate.

Referring to FIG. 10, the blood flow data (e.g., first blood flow data RAF_{Vasodilator}, second blood flow data RAF_{RDN} and base blood flow data RAF_{basal}) within the renal artery may be acquired by measuring impedance using the electrode 115 of the catheter. Alternatively, in another embodiment, the blood flow data (e.g., first blood flow data RAF_{Vasodilator}, second blood flow data RAF_{RDN} and base blood flow data RAF_{basal}) within the renal artery may be acquired through impedance sensors IE1, IE2, IE3 disposed adjacent to the electrode 115 of the catheter.

Also, the impedance sensor may detect changes in blood flow impedance, from which the blood flow speed may be acquired.

Referring to FIG. 11, the blood flow data (e.g., first blood flow data RAF_{Vasodilator} second blood flow data RAF_{RDN} and base blood flow data RAF_{basal}) within the renal artery may be acquired by an optical sensor.

Specifically, the optical sensor may include a light emitting unit LE and a light receiving unit LR, and the light emitting unit LE and light receiving unit LR may be disposed below the electrode described above. Accordingly, when the electrode is extended toward the inner wall of the renal artery, the light emitting unit LE irradiates light to the blood inside the renal artery and the light receiving unit LR may receive the light reflected from the blood. The light emitting unit LE may be made of an element that emits light. For example, the light emitting unit LE may include a light emitting diode (LED) that is harmless to the human body. In addition, the light receiving unit LR may include an image sensor in which light emitted from the light emitting unit is reflected from blood cells (e.g., red blood cells, RBC) or receives a diffraction pattern or shadow. The image sensor may include a CMOS image sensor.

The above-described method of acquiring blood flow data may be equally applied to acquiring the first blood flow data RAF_{Vasodilator} second blood flow data RAF_{RDN} and base blood flow data RAF_{basal} in the present specification.

Referring to FIG. 7, a method for evaluating an outcome of renal denervation according to another embodiment may comprise the steps of acquiring first blood flow data from a first region in a renal blood vessel after a vasodilator injected into the first region is activated in a second region in the renal blood vessel, acquiring base blood flow data in the first region (renal artery), and acquiring second blood flow data from the first region after renal denervation is performed with a renal denervation catheter.

More specifically, the method for evaluating an outcome of renal denervation may comprise the steps of injecting a vasodilator and activating the vasodilator (S3110), acquiring first blood flow data from a first region (renal artery) (S3120), acquiring base blood flow data (S3130), performing renal denervation with a catheter located in the first region (renal artery) (S3140), acquiring second blood flow data from the first region (renal artery) after performing the renal denervation with a catheter located in the first region (renal artery) (S3150) and comparing the first blood flow data and the second blood flow data (S3160). That is, unlike the description with respect to the previous embodiments, the step of acquiring base blood flow data in the renal artery may be performed between the step of injecting a vasodilator and the step of performing the renal denervation with a catheter located within the renal artery.

Further, except for the details described later, the step of injecting a vasodilator and activating the vasodilator (S3110) may be performed in the same way as the step of injecting a vasodilator and activating the vasodilator (S1110, S2120) described above, the step of acquiring first blood flow data from a first region (renal artery) (S3120) may be performed in the same way as the step of acquiring the first blood flow data from a first region (renal artery) (S1 120, S2130) described above, the step of acquiring base blood flow data (S3130) may be performed in the same way as the step of acquiring base blood flow data (S2110) described above, the step of performing renal denervation with a catheter located in the first region (renal artery) (S3140) may be performed in the same way as the step of performing renal denervation with a catheter located in the first region (renal artery) (S 1130, S2140) described above, the step of acquiring second blood flow from the first region (renal artery) after performing the renal denervation (S3150) may be performed in the same way as the step of acquiring second blood flow data from the first region (renal artery) after performing the renal denervation (S1140, S2150) described above, and the step of comparing the first blood flow data and the second blood flow data (S3160) may be performed in the same way as the step of comparing the first blood flow data and the second blood flow data (S1150, S2160).

In this embodiment, the vasodilator is characterized by a sufficiently short half-life so as not to affect the base blood flow data acquisition step. For example, the vasodilator is injected into the first region and activated in the second region, and the base blood flow data or second blood flow data may be acquired after the half-life of the vasodilator has elapsed. In other words, the base blood flow data or second blood flow data may be acquired after the half-life of the vasodilator has elapsed after the vasodilator is injected into the first region or second region. As will be described later, when the base blood flow data is acquired before the first blood flow data, the base blood flow data may be acquired regardless of the half-life. However, if the second blood flow data or base blood flow data is acquired after the first blood flow data is acquired, the second blood flow data or base blood flow data may be acquired after the half-life has elapsed after injection of the vasodilator. In addition, in an embodiment, the vasodilator is injected into the first region, but is provided to the second region according to movement, and it should be understood that this is expressed as 'injection'. In addition, since the vasodilator quickly moves to the second region after being injected into the first region, the base blood flow data or second blood flow data may be acquired after the half-life has elapsed after the vasodilator moves to the second region.

The step of acquiring base blood flow data (S3130) may be performed a predetermined time after the vasodilator is injected. In other words, the vasodilation of the renal arterioles, which is a state similar to the state of renal denervation due to the vasodilator, may return to the state before the vasodilator was administered (all recovered) after a predetermined period of time. According to this embodiment, there is only an acquisition time difference between the first blood flow data and the base blood flow data, and as described above, it is desirable that there is no change in the position of the catheter, etc.

That is, the first blood flow data, second blood flow data, and base blood flow data may be acquired while the driving tube is moved distally or proximally, as described above. For example, the first blood flow data, second blood flow data, and base blood flow data may be acquired while the driving tube is moved to either the distal or proximal side. In other words, the first blood flow data, second blood flow data, and base blood flow data are acquired while the catheter in the renal artery is in the same state, so that errors in each blood flow data may be minimized.

In addition, the first blood flow data, second blood flow data, and base blood flow data may be acquired by the blood flow information detected in the first region in the renal artery by the above-described detection means. For example, the first blood flow data, second blood flow data, and base blood flow data may be acquired while the position of the driving tube is the same. In other words, the operator moves the driving tube in the longitudinal direction by manipulating the proximal end of the drive tube, and while manipulating the proximal end of the drive tube by the same amount to move the driving tube by the same amount in the longitudinal direction, and thus may acquire the first blood flow data, second blood flow data, and base blood flow data in this state. Accordingly, when the catheter is disposed at the same position within the renal blood vessel, particularly within the first region, each of the first blood flow data, second blood flow data, and base blood flow data may be acquired so that an accurate evaluation of renal denervation may be performed.

FIG. 17 is a block diagram of a device for evaluating an outcome of renal denervation according to an embodiment.

Referring to FIG. 17, the renal denervation outcome evaluation device 130 according to an embodiment may comprise a first input unit 131, a second input unit 132, a third input unit 133, a first calculation unit 134, and a first calculation unit 134, a second calculation unit 135, and an evaluation unit 136.

The renal denervation outcome evaluation device 130 according to the embodiment may be configured to include a first input unit 131 that inputs first blood flow data from a first region in a renal blood vessel after a vasodilator is injected into the first region, and a second input unit 132 that inputs second blood flow data after performing renal denervation using a catheter in the first region.

The device may further include a third input unit 133 that inputs base blood flow data from the first region before injecting the vasodilator.

Blood flow information may be detected from a detection means disposed at the distal end of the catheter. As described above, the blood flow information includes blood flow volume, blood flow speed, blood flow resistance, etc., and the acquisition means may acquire or receive any one of the blood flow information from the detection means. For example, the first blood flow data, second blood flow data, and base blood flow data acquired by the acquisition means may belong to the same category of blood flow information, for example, blood flow volume. Also, the detection means may include any one of an ultrasonic sensor, an impedance sensor, and an optical sensor. In addition, as described above, the acquisition means may acquire the first blood flow data, second blood flow data, and base blood flow data based on the blood flow information detected by the detection means. The catheter may include the acquisition means described above. Further, the catheter may include a vasodilator injection unit.

First, the first input unit 131 may receive the first blood flow data from the first region (renal artery). For example, the first input unit 131 may be connected to the above-described detection means to receive the blood flow data acquired from the acquisition means. In this case, the detection means is connected to the acquisition means as described above, measures the blood flow information measured by the detection means and transmits it to the acquisition means, and the acquisition means may acquire the blood flow data by processing the measured blood flow information. This detection means includes an ultrasonic sensor, etc., and acquires blood flow data (e.g., first blood flow data, second blood flow data, and base blood flow data) measured in the first region (renal artery) and may receive the blood flow data acquired by the acquisition means. Accordingly, hereinafter, the second input unit 132 and third input unit 133, like the first input unit 131, are connected to the acquisition means and may receive the blood flow data acquired by the acquisition means to evaluate renal denervation.

For example, the first input unit 131 may receive the first blood flow data, which is blood flow data in the first region (renal artery), from the acquisition means within a predetermined time after injecting the vasodilator, and input it for evaluation of an outcome of the renal denervation.

The second input unit 132 may receive the second blood flow data in the first region (renal artery). That is, the second input unit 132 may receive the second blood flow data, which is blood flow data in the first region after performing renal denervation using a catheter, to evaluate the renal denervation. In addition, the second input unit 132 is connected to the above-described detection means and may receive the blood flow data acquired from the acquisition means. In this case, the detection means may include an ultrasonic sensor, etc. as described above.

The third input unit 133 may receive the base blood flow data, which is blood flow data in the first region (renal artery) before injecting the vasodilator or performing the renal denervation, to evaluate the renal denervation. For example, the third input unit 133 may receive the base blood flow data received from the acquisition means before injecting the vasodilator. Alternatively, the third input unit 133 may receive the base blood flow data received from the acquisition means after a predetermined time has elapsed after the vasodilator is injected and the first blood flow data is input.

As described above, the renal denervation may be evaluated even if the base blood flow data and first blood flow data are performed once, unlike the second blood flow data that may be input multiple times. Accordingly, in the renal denervation outcome evaluation device according to an embodiment, there is no need to repeatedly acquire the first blood flow data and base blood flow data, which are references for evaluation of renal denervation, so the procedure time may be reduced and the outcome of ablation may be evaluated without the complex compounds of vascular due to renal denervation.

Furthermore, as described above, the first input unit 131 to third input unit 133 may receive the blood flow data including blood flow rate and blood flow volume in the renal artery from the acquisition means, and receive the data from the same device with a predetermined time difference, and then input the data into each calculation unit to be described later.

The first calculation unit 134 may calculate a ratio between the first blood flow data and the second blood flow data. Also, the second calculation unit 135 may calculate a ratio between the first difference value and the second difference value. As described above, here, the first difference value may be a difference value between the first blood flow data and the base blood flow data, and the second difference value may be a difference value between the second blood flow data and the base blood flow data. In an embodiment, the first calculation unit 134 and second calculation unit 135 may calculate the above-mentioned ratio or proportion using the first blood flow data, second blood flow data, and base blood flow data input from the first input unit 131 to the third input unit 133.

The evaluation unit 136 may receive the ratio or proportion calculated from the first calculation unit 134 and second calculation unit 135 and use the ratio or proportion to evaluate the renal denervation.

More specifically, the evaluation unit 136 may compare the first blood flow data and the second blood flow data. For example, the evaluation unit 136 may compare the ratio or proportion of the first blood flow data and second blood flow data with a threshold value (e.g., a first threshold value). For example, it may be determined whether the ratio of the second blood flow data and first blood flow data is lower or higher than a first threshold. Alternatively, it may be determined whether the ratio of the second blood flow data and first blood flow data is lower or higher than a predetermined ratio. For example, when the ratio between the second blood flow data and the first blood flow data is lower than the first threshold, the evaluation unit 136 may evaluate that renal denervation should be performed again. Accordingly, information or an alarm that renal denervation must be re-performed may be output to the operator by the output unit. In addition, when the ratio between the second blood flow data and the first blood flow data is higher than the first threshold, the evaluation unit 136 may evaluate that renal denervation has been performed correctly. Accordingly, the evaluation unit 136 may provide an alarm about the completeness of the ablation to the operator through the output unit indicating that the renal denervation has been appropriately performed.

In addition, the evaluation unit 136 compares the ratio or proportion between the first difference value and the second difference value with a threshold value, and may determine whether the ratio between the first difference value and the second difference value (e.g., corresponding to the 'PEI' described above) is greater than or lower than a threshold value (e.g., a second threshold value).

For example, the evaluation unit 136 may evaluate that renal denervation should be performed again when the ratio between the first difference value and the second difference value is lower than the second threshold value. Accordingly, information or an alarm that renal denervation must be re-performed may be output to the operator by the output unit.

The evaluation unit 136 may evaluate that renal denervation has been performed correctly when the ratio between the first difference value and the second difference value is higher than the first threshold, and then transmit an alarm about the completeness of the ablation to the operator through the output unit.

The output unit (not shown) may output or provide an output (e.g., an alarm) corresponding to the evaluation of the outcome of the renal denervation to the user. The output unit (not shown) may provide the user with the adequacy or evaluation of renal denervation through various output methods such as a display. For example, the output unit (not shown) includes a display unit, and the evaluation outcomes (a ratio between the first difference value and the second difference value, a ratio between the first blood flow data and the second blood flow data, etc.) of the evaluation unit may be displayed on the display unit. In addition, the output unit (not shown) may display at least one of the first blood flow data, second blood flow data, and base blood flow data. For example, the output unit (not shown) may output the above-described peak-to-peak value, average value, or first blood flow data, second blood flow data, and base blood flow data acquired over a predetermined time in real time.

Further, if the evaluation unit 136 determines that the second blood flow data is lower than a predetermined proportion of the first blood flow data or the ratio between the first difference value and the second difference value is lower than a threshold as described above, the output unit (not shown) may provide the user with information that renal denervation is incomplete. For example, an output unit (not shown) may display that renal denervation is not complete (corresponding to renal denervation being re-performed) or provide a specific sound to the user. In this case, the specific sound may be different from the sound output when renal denervation is completed. In addition, an output unit (not shown) may provide the user with completion of renal denervation. That is, according to the determination of the evaluation unit 136, the output unit (not shown) may output the evaluation of renal denervation or provide an alarm to the operator.

A term of 'unit' used in the embodiment means software or a hardware component such as a field-programmable gate array (FPGA) or ASIC, and 'unit' performs predetermined roles. However, 'unit' is not a meaning limited to software or hardware. 'Unit' may be configured to be positioned in an addressable storage medium and configured to regenerate one or more processors. Therefore, as one example, 'unit' includes components such as software components, object oriented software components, class components, and task components, processes, functions, attributes, procedures, subroutines, segments of a program code, drivers, firmware, a microcode, a circuit, data, a database, data structures, tables, arrays, and variables. Functions provided in the components and 'units' may be joined as a smaller number of components or further separated into additional components and 'units'. The components and 'units' may be implemented to regenerate one or more CPUs within a device or a security multimedia card.

While the present invention is described mainly based on the above embodiments but is not limited thereto, it will be understood by those skilled in the art that various changes and modifications are made without departing from the spirit and scope of the present invention. For example, each component specifically shown in the embodiments may be modified and implemented. It should be interpreted that differences relating to such modifications and application are included in the scope of the present invention defined in the appended claims.

## Claims

1. A method for evaluating an outcome of renal denervation comprising the steps of:
acquiring first blood flow data from a first region in a renal blood vessel after a vasodilator injected into the first region is activated in a second region in the renal blood vessel; and
acquiring second blood flow data from the first region after renal denervation is performed with a catheter for denervation.

2. The method of claim 1, further comprising the step of comparing the first blood flow data and the second blood flow data.

3. The method of claim 2, wherein the renal denervation is evaluated according to a result of the comparison of the first blood flow data and the second blood flow data.

4. The method of claim 3, wherein if the comparison result is lower than a predetermined threshold, the step of re-performing renal denervation is repeated.

5. The method of claim 1, further comprising the steps of:
calculating a first difference value representing a difference value between the first blood flow data and base blood flow data;
calculating a second difference value representing a difference between the second blood flow data and the base blood flow data; and
comparing the first difference value and the second difference value.

6. The method of claim 5, wherein an outcome of the renal denervation is evaluated according to a result of the comparison of the first difference value and the second difference value.

7. The method of claim 6, wherein if the comparison result is lower than a predetermined threshold, the step of re-performing renal denervation is repeated.

8. The method of claim 5, wherein the base blood flow data is acquired before injecting the vasodilator, or between the step of acquiring the first blood flow data and the step of performing the renal denervation.

9. The method of claim 1 or 5, wherein base blood flow data or the second blood flow data is acquired when a half-life has elapsed after the vasodilator is injected into the first region.

10. The method of claim 1, wherein the second region is a renal arteriole or renal capillary.

11. The method of claim 1, wherein the vasodilator includes any one of dopamine, dobutamine, adenosine, prostacyclin, nitric oxide, bradykinin, papaverine, dipyridamolum, diuretin, theophylline, minoxidil, and isosorbide.

12. A device for evaluating an outcome of renal denervation comprising:
a first input unit that inputs first blood flow data acquired from a first region in a renal blood vessel after a vasodilator injected into the first region is activated in a second region; and
a second input unit that inputs second blood flow data acquired from the first region after renal denervation is performed with a catheter.

13. The device of claim 12, further comprising a first calculation unit that calculates a ratio between the first blood flow data input by the first input unit and the second blood flow data input by the second input unit.

14. The device of claim 13, further comprising an evaluation unit that evaluates an outcome of renal denervation by comparing the ratio between the first blood flow data and the second blood flow data calculated by the first calculation unit with a first threshold value.

15. The device of claim 13, further comprising a third input unit that inputs base blood flow data from the first region before injection of the vasodilator.

16. The device of claim 15, further comprising a second calculation unit that calculates a ratio between a first difference value and a second difference value,
wherein the first difference value is a difference value between the first blood flow data and the base blood flow data, and the second difference value is a difference value between the second blood flow data and the base blood flow data.

17. The device of claim 16, further comprising an evaluation unit that evaluates the outcome of renal denervation by comparing the ratio between the first difference value and the second difference value calculated by the second calculation unit with a second threshold value.

18. The device of claim 14 or 17, further comprising a display unit that displays an evaluation result of the evaluation unit.

19. The device of claim 14 or 17, further comprising an output unit that outputs an audio signal or video signal, etc., according to the evaluation result of the evaluation unit.

20. The device of claim 12 or 15, wherein the first blood flow data, the second blood flow data, and the base blood flow data are acquired from blood flow information detected by a detection means disposed on the catheter.

21. The device of claim 20, wherein the blood flow information includes at least one of blood flow volume, blood flow rate, and blood flow resistance.

22. The device of claim 20, wherein the detection means includes any one of an ultrasonic sensor, an impedance sensor, and an optical sensor.

23. The device of claim 22, wherein the ultrasonic sensor includes a Doppler sensor.

24. The device of claim 22, wherein an impedance value is detected by an electrode of the catheter or detected by a separate sensor.

25. The device of claim 22, wherein the catheter includes a vasodilator injection unit.
